# EUROPÄISCHE PATENTSCHRIFT

(11) **EP 0 553 782 B1**
(45) Veröffentlichungstag und Bekanntmachung des Hinweises auf die Patenterteilung: **02.04.1997**
(21) Anmeldenummer: 93101193.6
(22) Anmeldetag: 27.01.1993
(51) Int. Cl.: A61B 17/56

(54) **Wirbelsäulenimplantat und Repositionsinstrument**
Rachidial implant and reposition instrument
Implant rachidien et instrument de reposition

(30) Priorität: 31.01.1992 DE 4202748
(43) Veröffentlichungstag der Anmeldung: 04.08.1993
(73) Patentinhaber: Kluger, Patrick, Dr. med., D-89155 Erbach (DE)
(72) Erfinder: Kluger, Patrick, Dr. med., D-89155 Erbach (DE)
(74) Vertreter: Patentanwälte Eisele, Otten & Roth

(56) Entgegenhaltungen:
- EP-A- 0 425 783
- DE-A- 3 711 091
- US-A- 4 987 892

## Beschreibung

Die Erfindung betrifft ein Implantat zur Stabilisierung der Wirbelsäule mit einem Längsträger in Form eines Profilstabes, an diesen anklemmbaren Querauslegern und mit an den Querauslegern mit einstellbarer Neigung befestigbaren Verankerungsschrauben.

Bei allen bekannten Fixationssystemen dieser Art werden zwei Längsträger verwendet, die im wesentlichen symmetrisch zur Wirbelsäulenlängsachse angeordnet sind. Dies gilt insbesondere bei kurzstreckigen, d. h. zur Überbrückung nur eines Wirbelkörpers bestimmten Implantaten, wie z. B. nach DE-A-32 19 575 oder nach dem Beitrag von P. Kluger aus TH. Stuhler, "Fixateur extern - Fixateur intern", Springer-Verlag Berlin Heidelberg 1989, Seiten 36 bis 58.

Auch die US-A-4 987 892 zeigt ein Implantat der einleitend bezeichneten Art. Es besteht aus einem querschnittlich kreisrunden Profilstab, an dem 2 Klemmstücke angesetzt sind, deren Backen jeweils mittels einer durchgehenden Schraube mit dem flachen Kopf einer Verankerungsschraube zusammengespannt sind. Somit ist der Abstand des Kopfs der Verankerungsschraube vom Profilstab nicht variabel, was zur Folge hat, daß nur zwei Verankerungsschrauben mittels eines Profilstabes miteinander verbunden werden können, es sei denn, eine weitere Verankerungsschraube stünde mit den anderen in einer geraden Linie.

Der Erfindung liegt die Aufgabe zugrunde, eine größere Distanz zu überbrücken und mehr als nur zwei Wirbelkörper zu fixieren, d. h. ein Implantat zu schaffen, mit dem auch langstreckige mehrsegmentale Instabilitäten und Fehlstellungen behandelt werden können.

Diese Aufgabe wird erfindungsgemäß dadurch gelöst, daß die Klemmstücke und die Verbinder ineinandergeschraubt sind, und daß die Verankerungsschrauben je einen abgeflachten Schraubenkopf haben, auf den je ein Verlängerungsstab mit einer wannenförmigen Aussparung seines verdickten Endabschnitts aufsteckbar ist. Hierbei ist nur ein einziger Längsträger erforderlich, der einfach auf die benötigte Länge gekürzt wird. Die Zahl der Querausleger und ihre Anordnung am Längsträger ist beliebig. Wie noch gezeigt wird, ist die Länge der einzelnen Querausleger kleinstufig wählbar und außerdem ist Vorsorge getroffen, daß auch zwei Verankerungsschrauben eines Wirbelkörpers, die in bekannter Weise mit deutlicher Konvergenz durch die Bogenwurzeln eingeschraubt sind, an dem Längsträger angebracht werden können, ohne daß sich die entsprechenden Querausleger gegenseitig stören.

Dieses baukastenmäßig variabel zusammenfügbare Implantat dient zur bleibenden Fixierung einer im Prinzip beliebigen Anzahl von Wirbeln zueinander in ihrer mittels der Verlängerungsstäbe und eines geeigneten Repositionsinstrumentariums erzielten Stellung. Danach kann durch angelagerte Knochensubstanz eine Versteifung des instrumentierten Wirbelsäulenabschnitts eintreten. Das Implantat paßt sich der unterschiedlichen örtlichen Lage, Distanz und räumlichen Richtung der Verankerungsschrauben perfekt an. Bei hinreichender Stabilität bleibt außerdem die Menge implantierten Materials verhältnismäßig klein.

Die Bohrungsachse der Rasterfläche des Verbinders muß senkrecht zu dessen Gewindeschaft stehen. Das Klemmstück hat zweckmäßigerweise zwei zangenartige Backen, die eine passende Aufnahmeöffnung für den Profilstab bilden. Bei einer Ausführungsform als Ringschelle sind die beiden Backen am Klemmstück selbst angeformt, wobei die Klemmbewegung sich im Rahmen der Werkstoffelastizität vollzieht. Das Klemmstück kann aber auch mit Vorteil zweiteilig ausgebildet sein, wobei ein Backen scharnierartig angelenkt ist. Solche Klemmstücke brauchen nicht auf den Stab aufgefädelt zu werden. Man kann sie unmittelbar am Längsträger ansetzen oder nachträglich zwischen bereits montierte Klemmstücke einfügen. Die Schraubverbindung zwischen Klemmstück und Verbinder erfolgt vorzugsweise so, daß das Klemmstück an der gegenüberliegenden Seite der Aufnahmeöffnung für den Profilstab als Schraubhülse ausgebildet ist, also eine Gewindebohrung aufweist, die sich vorzugsweise senkrecht zur Aufnahmeöffnung erstreckt. Der Verbinder ist in diesem Fall gegliedert in eine vorzugsweise runde Scheibe mit Rasterfläche mit einem radial ansitzenden Gewindeschaft.

Zur Längenanpassung werden die Verbinder mit Gewindeschäften geeigneter Längenabstufung bereitgehalten. Durch entsprechende Neigung des Querauslegers kann dieser auf die Höhe des Kopfs der Verankerungsschraube eingestellt und durch Spannen der Klemmschraube festgelegt werden. Die Richtung der Verankerungsschraube wird in der einen Achse durch die Winkelstellung des Gewindezapfens und in der anderen Achse durch die relative Einstellung der Rasterscheiben erfaßt.

Um große Neigungen der Querausleger zu ermöglichen, ohne daß ihre klemmschraubenseitigen Teile nach außen überstehen, wird vorgeschlagen, daß das Klemmstück eine geknickte Form hat, so daß die Achsen der Gewindebohrung und der Klemmschraube schiefwinklig zueinander stehen. Um ferner zwei Klemmstücke ohne Behinderung möglichst eng aneinanderrücken und dadurch beide Verankerungsschrauben eines Wirbelkörpers aufnehmen zu können, wird vorgeschlagen, daß die Achsen der Aufnahmebohrungen für den Verbinder einerseits und für die Klemmschraube andererseits am Klemmstück in Richtung des Profilstabs gegeneinander versetzt sind.

Dem nachfolgend vorgeschlagenen Repositionsinstrumentarium liegt z. B. bei einer seitlichen Ausbiegung der Wirbelsäule folgende Problemstellung zugrunde. Um den betroffenen Wirbelsäulenabschnitt zurückzubiegen, müssen auf die Wirbel oder wenigstens auf einige von Ihnen zur konkaven Seite der Biegung gerichtete Zugkräfte aufgebracht werden. Diese erfordern ein stabiles Gegenlager außerhalb der Wunde und geeignete Traktionsinstrumente.

Bekannt sind aus DE-A-34 14 374 sowie DE-A-37 11 091 nach ihrem Verwendungszweck so bezeichnete Distraktions- bzw. Kompressionsinstrumente. Sie dienen dazu, zwischen zwei Verankerungsschrauben, die an abnehmbaren Verlängerungsstäben gefaßt werden, eine mechanische Verbindung herzustellen und zwar außerhalb der Wunde. Dabei bleibt der instrumentierte Wirbelsäulenabschnitt intraoperativ zugänglich. Durch Verschieben des beweglichen Armes kann zwischen den beiden Wirbeln, in denen die Verankerungsschrauben sitzen, eine Druck- oder Zugkraft ausgeübt werden. Es wird vorgeschlagen, ein solches aus einem Längsholm sowie einem festen und einem in Längsrichtung verstellbaren Arm bestehendes Instrument, an dessen Armen je ein Träger eines Aufnahmehohlkörpers für einen Verlängerungsstab angelenkt ist, dadurch weiterzubilden, daß die Gelenkachsen etwa in der durch die Arme definierten Ebene und derart schräg verlaufen, daß sie sich in der Verlängerungsrichtung auf der dem Längsholm zugewandten Seite kreuzen. Diese Gelenkanordnung erlaubt es, den Längsholm als Angriffsbasis für die oben erwähnten Zugkräfte zu verwenden, sofern der Längsholm nur lang genug ist, um eine Brücke zwischen dem oberen und dem unteren Wirbelkörper, die den betroffenen Wirbelsäulenabschnitt abschließen, zu bilden.

Die Gelenkachsen können zweckmäßigerweise einen Winkel von etwa 30° bezüglich des Längsholms aufweisen. Um die Angriffspunkte der Traktionsinstrumente ausreichend zu fixieren, wird weiter vorgeschlagen, daß der Längsholm des vorerwähnten Instruments eine Zahnstange ist, deren Zähne an der von den Armen abgewandten Seite liegen und insbesondere hinsichtlich ihres Abstandes so beschaffen sind, daß an den Traktionsinstrumenten angebrachte Haken zwischen die Zähne eingelegt werden können.

Die Zugkräfte zur Reposition der Zwischenwirbel müssen unter Beibehaltung der räumlichen Winkelstellung der Verankerungsschrauben an diesen angreifen. Um dies zu erreichen wird als Traktionsinstrument ein Repositionsinstrument vorgeschlagen, das zwei von einer Spindel durchsetzte und durch deren Schraubbewegung zusammenziehbare Hülsen aufweist, von denen die eine einen Haken und die andere einen zur Zugrichtung schrägen Arm aufweist, an dem ein Aufnahmehohlkörper für einen Verlängerungsstab so angebracht ist, daß er um eine zur Gewindespindel senkrechte Achse einstell- und fixierbar ist. Die erforderlichen Freiheitsgrade für die Anpassung an die Richtung der Verankerungsschraube ergeben sich durch die freie Drehbarkeit des schrägen Armes um die Spindelachse, durch die freie Drehbarkeit des Verlängerungsstabes im Aufnahmehohlkörper und durch die Einstellbarkeit des Aufnahmehohlkörpers bezüglich des Armes.

Bei diesem Repositionssystem bleiben die Vorteile der bekannten kurzstreckigen Instrumentierung erhalten, nämlich die Umlenkung der mechanischen Verbindung während der Operation nach außerhalb der Wunde, die leicht abnehmbaren Verlängerungsstäbe der einzelnen Verankerungsschrauben als Repositionshebel und die winkelstabile Verbindung dieser Hebel mit den Armen bzw. Zugspindeln der Instrumente. Bei dieser letzteren Verbindung bleibt die Stellungskorrektur in allen Freiheitsgraden erhalten.

Das Dauerimplantat ist vorteilhaft schlank und flach, so daß Montagen auch an der oberen und mittleren Brustwirbelsäule möglich sind. Weitere Einsatzgebiete für die vorgeschlagenen Implantate und Instrumente sind polysegmentale Zerstörungen bei Tumoren, manche Serienfrakturen, degenerative Veränderungen und ganz besonders Skoliosen im Sinne einer dorsal derotierenden Spondylodese (DDS).

Ausführungsbeispiele der Erfindung werden nachfolgend anhand der Zeichnung beschrieben. Im einzelenen zeigt
- Fig. 1: den Teillängsschnitt eines Querauslegers nach der Schnittlinie I-I,
- Fig. 2: die Draufsicht eines Teils eines Implantats zur langstreckigen Wirbelsäulenfixierung,
- Fig. 3: den Längsschnitt eines kurzen einstückigen Klemmstücks,
- Fig. 4: den Längsschnitt eines zweiteiligen Klemmstücks in größerem Maßstab,
- Fig. 5: die Draufsicht des Klemmstücks nach Fig. 4,
- Fig. 6: die Ansicht eines kurzen Verbinders,
- Fig. 7: einen Teilschnitt des Verbinders nach Fig. 6,
- Fig. 8: die Seitenansicht eines Traktionsinstruments,
- Fig. 9: die Seitenansicht, teilweise geschnitten, eines speziellen Distraktionsinstruments, das die endständigen Wirbel verbindet und die Angriffsbasis für Traktionsinstrumente bildet,
- Fig. 10: die Stirnansicht des Distraktionsinstruments nach Fig. 9 und
- Fig. 11: die schematische Ansicht einer typischen Anordnung während der Operation.

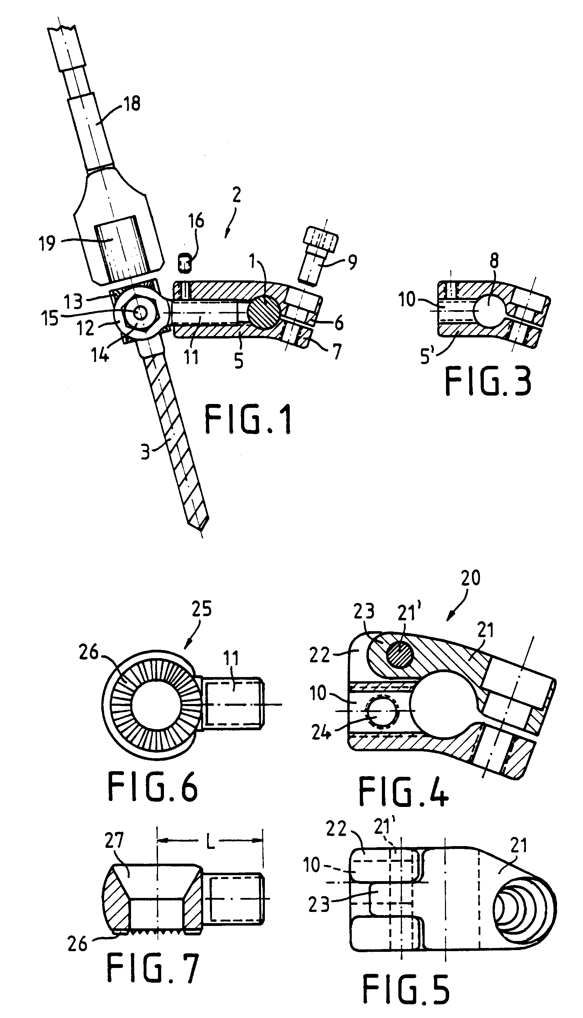

Wie die Figuren 1 und 2 darstellen, besteht das Implantat aus einem querschnittlich kreisrunden Profilstab 1 mit mehreren Querauslegern 2, an denen jeweils eine Verankerungsschraube 3 befestigt ist. Der Profilstab 1 wird zuvor auf die erforderliche Länge abgeschnitten und durch Biegen dem gewünschten Verlauf der Wirbelsäule angepaßt, um seitlich neben den Dornfortsätzen Platz zu finden. Die Wirbelkörper 4 des betrachteten Wirbelsäulenabschnitts sind schematisch angedeutet.

Die Querausleger sind mit ihren Klemmstücken 5 an dem Profilstab 1 angeklemmt. An den Klemmstücken sind dazu zwei Backen 6 und 7 angeformt, die eine kreiszylindrische Aufnahmeöffnung 8 (Fig. 3) für den Profilstab 1 bilden und eine Gewindebohrung aufweisen, um mittels einer Inbusschraube 9 zusammengespannt zu werden, wobei die Aufnahmeöffnung 8 infolge elastischer Verformung so verengt wird, daß das Klemmstück 5 fest auf dem Profilstab 1 haftet. Auf der anderen Seite befindet sich in dem Klemmstück 5 und dessen anderen Ausführungsformen eine Gewindebohrung 10 zur Aufnahme des Gewindeschafts 11 eines plattenförmigen Verbinders 12. Wie die Draufsichten gemäß Fig. 2 zeigen, ist die Gewindebohrung 10 gegenüber der Achse der Inbusschraube 9 in Richtung des Profilstabes 1 versetzt. Der Verbinder 12 hat auf einer Seite eine sternförmige Rasterfläche, die mit der Rasterfläche 13 am abgeflachten Kopf der Verankerungsschraube 3 zusammenwirkt und deren Winkelfestigkeit garantiert. Die Verbindung erfolgt mit Hilfe einer Sechskant-Kopfschraube 14, die als Montagehilfe eine zylindrische Haltebohrung 15 aufweist. Der Gewindeschaft 11 wird mittels einer Madenschraube 16 mit Innensechskant fixiert. Gleiche Sicherungsschrauben 17 für die Kopfschrauben 14 sind auch an der Stirnseite der Verankerungsschrauben vorgesehen.

Zum Einsetzen der Verankerungsschrauben 3 und als Hebel zum Aufbringen der Kräfte dient bekanntermaßen ein Verlängerungsstab 18, der mit der wannenförmigen Aussparung 19 seines verdickten unteren Endabschnitts auf den Schraubenkopf aufgesteckt und an diesem fixiert werden kann. Ein solcher Verlängerungsstab 18 ist in Fig. 1 angedeutet.

In Fig. 3 ist als Variante ein Klemmstück 5' dargestellt, dessen Gewindebohrung 10 gegenüber derjenigen beim Klemmstück 5 kürzer ist. Damit soll die Möglichkeit geschaffen werden, auch eine Verankerungsschraube mit sehr geringem Abstand an dem Profilstab 1 zu befestigen, wie in Fig. 2 unten rechts ersichtlich. Diese Partie zeigt übrigens auch, daß die beiden auf einer gemeinsamen Querachse liegenden Verankerungsschrauben eines Wirbelkörpers mit zwei Querauslegern 2 erfaßt werden können, wobei noch Platz zum Aufstecken der Kappe eines strichpunktiert angedeuteten Verlängerungsstabes 18 vorhanden ist.

Das Klemmstück 20 nach den Figuren 4 und 5 unterscheidet sich gegenüber den vorhergehenden durch einen gelenkig angebrachten Backen 21. Er hat eine zwischen zwei Höckern 22 des Klemmstücks angeordnete Nase 23, wobei ein Achsstift 21' die genannten Teile quer durchsetzt. Auch hier übernimmt eine Inbusschraube die Klemmfunktion. Die Gewindebohrung 10 für den Verbinder, die ebenfalls eine Querbohrung 24 für eine Sicherungsschraube aufweist, ist unterhalb des Scharniergelenks wiederum außermittig angeordnet.

Die Figuren 6 und 7 zeigen einen anderen Verbinder 25, der auf der seiner Rasterfläche 26 gegenüberliegenden Seite eine konische Vertiefung 27 zur Aufnahme des Kopfes einer nicht dargestellten Senkschraube mit Innensechskant aufweist. Der Gewindeschaft 11 ist im Beispiel extrem kurz. Ein Vorrat mit stufenweise größeren Längen L ist bereitzuhalten. Die versenkten Schrauben haben den Vorteil einer Verkleinerung und glatteren Oberfläche der Verbindungsanordnung.

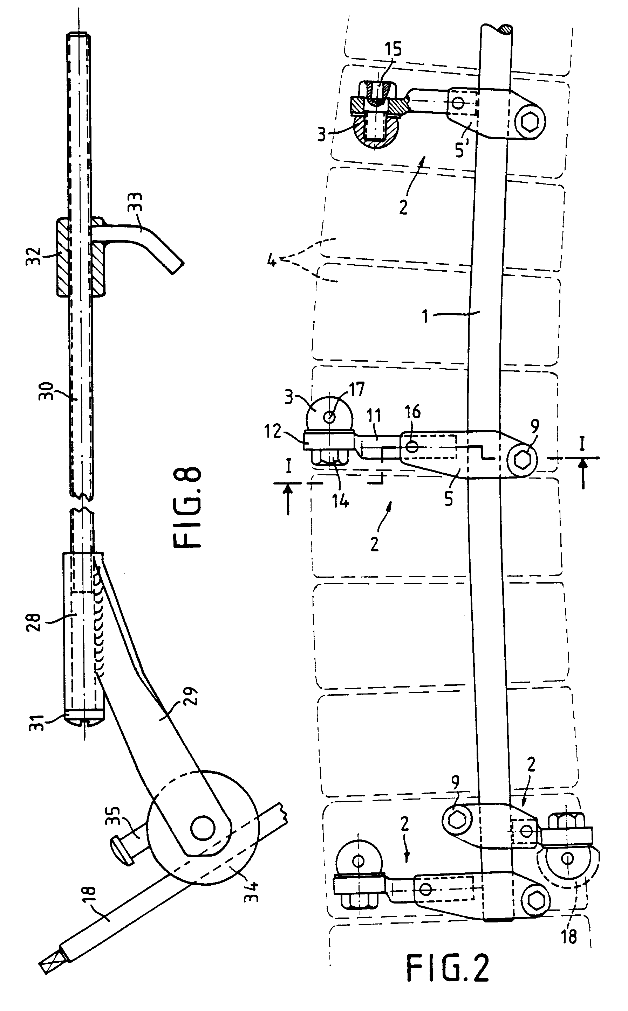

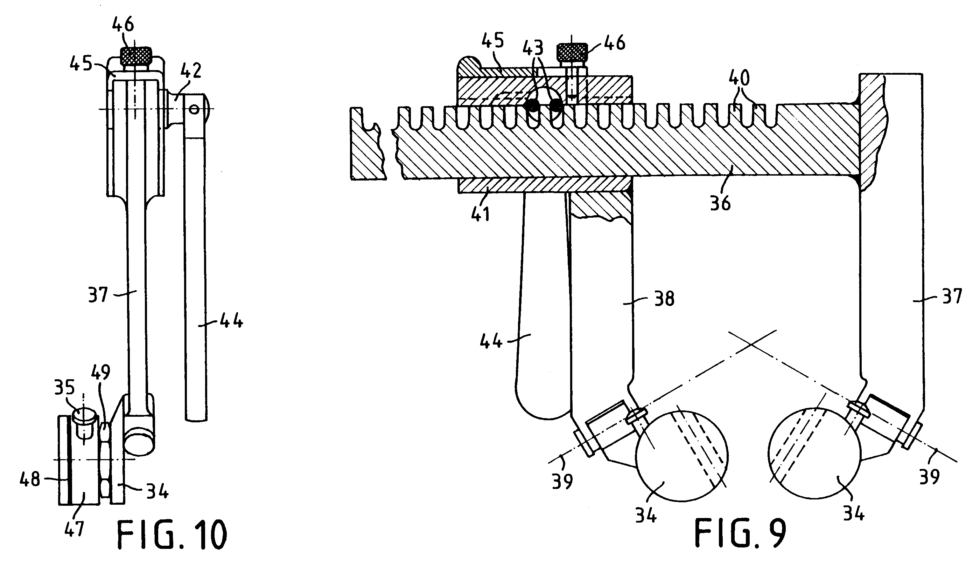

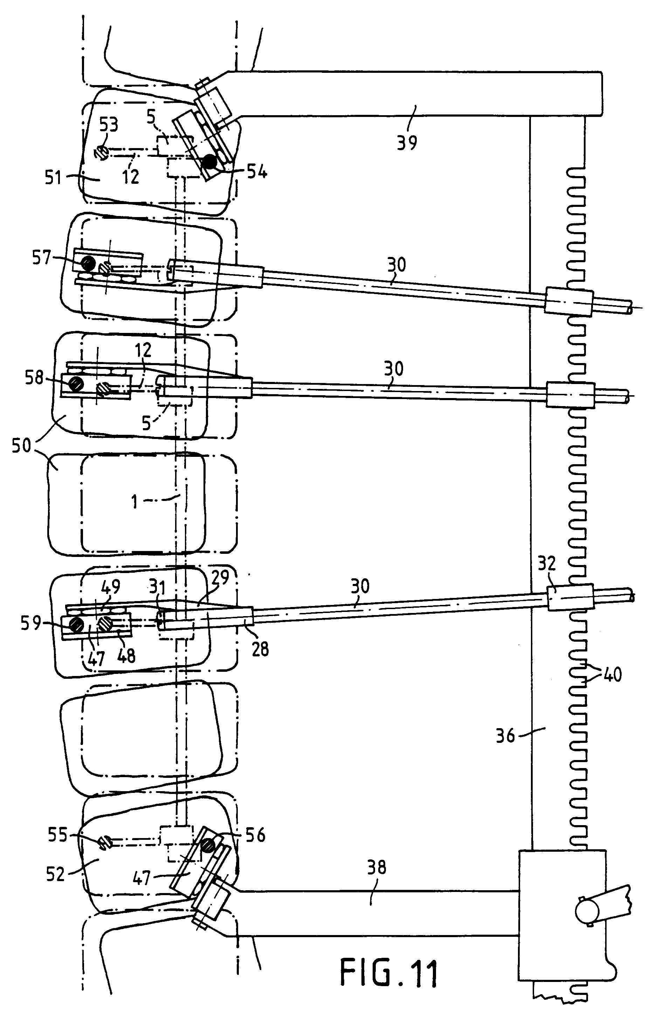

Das in Fig. 8 dargestellte Traktionsinstrument weist eine Hülse 28 mit glatter Bohrung und einen daran angeschweißten Schrägarm 29 auf. In die Hülse ist eine Gewindespindel 30 eingefügt, deren Kopf 31 stirnseitig anliegt. Auf die Spindel ist eine Gewindehülse 32 aufgeschraubt, die einen stabilen Haken 33 aufweist. An dem Schrägarm 29 ist der scheibenförmige Träger 34 eines bekannten zylinderscheibenförmigen Aufnahmehohlkörpers 47 befestigt. Letzterer kann mittels zusammenwirkender Rasterflächen 48 und einer diese arretierenden Ringmutter 49 (Figuren 10 und 11) in Winkelschritten von 6° bezüglich der Achse des Trägers 34 eingestellt werden. Gezeigt ist ferner das obere Ende eines vorerwähnten Verlängerungsstabes 18, der in der Tangentialbohrung des Aufnahmehohlkörpers in Längsrichtung verriegelt ist und durch Drücken einer Taste 35 gelöst werden kann. Dieses Instrument dient, wie noch erläutert wird, zum Aufbringen einer Zugkraft auf eine Verankerungsschraube.

Das Distraktionsinstrument nach den Fig. 9 und 10 besteht aus den Grundelementen Längsholm 36, fester Arm 37 und verschiebbarer Arm 38. An den Armen sind einfache einachsige Gelenke für die Träger 34 der Aufnahmehohlkörper 47 angeordnet. Ein wesentliches Merkmal dieses Instruments besteht darin, daß die Gelenkachsen 39 nicht fluchten, sondern schräg zueinander stehen und zwar so, daß sie sich in dem Bereich zwischen den Armen 37 und 38 kreuzen, die Träger 34 also einander zugewendet sind.

Der Längsholm 36 ist eine flache Zahnstange mit zinnenförmigen parallelflankigen Zähnen 40. Der verschiebbare Arm 38 ist mit einer Rechteckhülse 41 fest verbunden, in der eine Treibwelle 42 gelagert ist. Sie besteht im Dickenbereich der Zahnstange aus zwei parallelen Rundstiften 43, welche beim Drehen der Treibwelle abwechselnd in die Zahnlücken eintauchen. Als Betätigungsorgan zum Antrieb dient ein längerer Knebel 44. Mittels eines Schiebers 45 kann die Rechteckhülse 41 in einer bestimmten Stellung arretiert werden. Der Schieber seinerseits läßt sich mittels einer Arretierschraube 46 festlegen.

Fig. 11 zeigt schematisch in vollständig ausgezogenen Strichen die Wirbelkörper 50 eines während der Operation freigelegten, seitlich gekrümmten Wirbelsäulenabschnitts (Skoliose). Die oberen und unteren Wirbelkörper 51 und 52 sind die "neutralen" Abschlußwirbel des gekrümmten Bereichs. Sie sind mit dem Distraktionsinstrument gefaßt. Im Beispiel sind in jedem dieser beiden Wirbelkörper 51 und 52 zwei Verankerungsschrauben 53, 54 bzw. 55, 56 eingesetzt. Bei der Operation sind auf sämtliche Verankerungsschrauben, auch auf die im folgenden genannten, Verlängerungsstäbe 18 aufgesteckt. Die Verlängerungsstäbe der Schrauben 54 und 56 sind in die Aufnahmehohlkörper 47 des Distraktionsinstruments eingesteckt und festgehalten.

Ferner sind im Beispiel drei ausgewählte Wirbelkörper mit Verankerungsschrauben 57, 58 bzw. 59 versehen. An deren Verlängerungsstäben greifen drei Traktionsinstrumente, wie in Fig. 8 dargestellt, an. Deren Haken 33 sind über die kammförmige Zahnkante des Längsholms 36 des Distraktionsinstruments gehängt, das sich an der Konkavseite der Krümmung befindet.

Von dieser Situation ausgehend können unter begleitender Röntgenkontrolle und eventueller regulierender Einflußnahme auf die Winkelstellungen durch Drehen an den Schrauben 31 die Traktionsinstrumente verkürzt werden. Dadurch werden die verlagerten Wirbelkörper 50 in die gewünschte gerade Stellung gezogen, die strichpunktiert angedeutet ist. In dieser neuen, zunächst nur durch das Instrumentarium fixierten Stellung wird das Implantat montiert. Infolge der Schrägarme 29 liegen die Gewindespindeln 30 der Traktionsinstrumente so hoch, daß der Zugang nicht behindert ist. Der Längsstab 1, die Klemmstücke 5 und die Verbinder 12 der Querausleger sind in ihrer bleibenden Endstellung ebenfalls strichpunktiert angedeutet. Insgesamt sind dann im Beispiel fünf Wirbelkörper über sieben Verankerungsschrauben miteinander verbunden. Nach der Montage werden das Instrumentarium und zuletzt die Verlängerungsstäbe 18 abgenommen.

Die Zahl der Verankerungspunkte in dem instrumentierten Wirbelsäulenabschnitt richtet sich nach dem Grad der Instabilität. So werden z. B. bei Brüchen oder bei tumorösen Zerstörungen einzelner Wirbel viele Verankerungspunkte gebraucht, während andererseits eine an sich intakte, jedoch verkrümmte Wirbelsäule mit weniger Verankerungspunkten in ihrer Stellung gehalten werden kann. Entsprechend muß auch darüber entschieden werden, ob jeweils beide Bogenwurzeln der instrumentierten Wirbel zwischen dem oberen und unteren Abschlußwirbel mit Verankerungsschrauben gefaßt werden. Die Zahl der Wirbel, an denen Traktionsinstrumente angreifen müssen, richtet sich auch nach der Art, dem Ausmaß und der Rigidität der Fehlstellung. Schließlich ist es möglich, zusätzlich zu einem gem. Fig. 11 an der Konkavseite montierten Distraktionsinstrument eine Verbiegung der Wirbelsäule im gleichen Abschnitt in Richtung nach vorne oder hinten durch ein auf der Gegenseite zwischen weiteren Wirbeln montiertes Instrumentarium entsprechend zu beeinflussen. Derart mehrbogige, häufig doppelbogige Wirbelsäulenverkrümmungen treten nicht selten auf. Sie können mit dem beschriebenen Instrumentarium folgerichtig angegangen werden.
- 1: Profilstab
- 2: Querausleger
- 3: Verankerungsschraube
- 4: Wirbelkörper
- 5: Klemmstück
- 5': Klemmstück
- 6: Backen
- 7: Backen
- 8: Aufnahmeöffnung
- 9: Inbusschraube
- 10: Gewindebohrung
- 11: Gewindeschaft
- 12: Verbinder
- 13: Rasterfläche
- 14: Kopfschraube
- 15: Haltebohrung
- 16: Madenschraube
- 17: Sicherungsschraube
- 18: Verlängerungsstab
- 19: Aussparung
- 20: Klemmstück
- 21: Backen
- 21': Achsstift
- 22: Höcker
- 23: Nase
- 24: Querbohrung
- 25: Verbinder
- 26: Rasterfläche
- 27: Vertiefung
- 28: Hülse
- 29: Schrägarm
- 30: Gewindespindel
- 31: Kopf
- 32: Gewinde
- 33: Haken
- 34: Träger
- 35: Taste
- 36: Längsholm
- 37: fester Arm
- 38: beweglicher Arm
- 39: Gelenkachse
- 40: Zahn
- 41: Rechteckhülse
- 42: Treibwelle
- 43: Rundstift
- 44: Knebel
- 45: Schieber
- 46: Arretierschraube
- 47: Aufnahmehohlkörper
- 48: Rasterfläche
- 49: Ringmutter
- 50: Wirbelkörper
- 51: Abschlußwirbelkörper
- 52: Abschlußwirbelkörper
- 53: Verankerungsschraube
- 54: Verankerungsschraube
- 55: Verankerungsschraube
- 56: Verankerungsschraube
- 57: Verankerungsschraube
- 58: Verankerungsschraube
- 59: Verankerungsschraube

## Patentansprüche

1. Implantat zur Stabilisierung der Wirbelsäule mit einem Längsträger in Form eines Profilstabes (1), an diesem anklemmbaren Querauslegern (2) und an dem Querauslegern mit einstellbarer Neigung befestigbaren Verankerungsschrauben (3), wobei die Querausleger (2) aus je einem Klemmstück (5, 20) und einem Verbinder (12, 25) bestehen und die Verbinder je eine Rasterfläche (26) mit einer zentralen Bohrung aufweisen, um eine Verankerungsschraube (3) winkelfest anzuschrauben, dadurch gekennzeichnet, daß die Klemmstücke und die Verbinder ineinandergeschraubt sind, und daß die Verankerungsschrauben (3) je einen abgeflachten Schraubenkopf haben, auf den je ein Verlängerungsstab (18) mit einer wannenförmigen Aussparung (19) seines verdickten Endabschnitts aufsteckbar ist.

2. Implantat nach Anspruch 1, dadurch gekennzeichnet, daß das Klemmstück zwei zangenartige Backen (6, 7) aufweist, die eine passende Aufnahmeöffnung (8) für den Profilstab (1) bilden und mittels einer Klemmschraube (9) zusammengezogen werden, und daß an der gegenüberliegenden Seite der Aufnahmeöffnung an dem Klemmstück eine Gewindebohrung (10) zum Einschrauben des Verbinders (12) senkrecht zur Aufnahmeöffnung (8) vorgesehen ist.

3. Implantat nach Anspruch 2, dadurch gekennzeichnet, daß ein Backen (21) an dem Klemmstück (20) scharnierartig angelenkt ist.

4. Implantat nach Anspruch 1, dadurch gekennzeichnet, daß das Klemmstück (5, 20) eine geknickte Form hat, so daß die Achse der Gewindebohrung (10) und der Klemmschraube (9) schiefwinklig zueinander stehen.

5. Implantat nach Anspruch 4, dadurch gekennzeichnet, daß die Achsen der Gewindebohrung (10) und der Klemmschraube (9) am Klemmstück in Richtung des Profilstabes (1) gegeneinander versetzt sind.

6. Implantatsystem, gekennzeichnet durch ein Implantat nach Anspruch 1 in Kombination mit einem Distraktionsinstrument mit einem Längsholm (36), einem daran bestigten Arm (37) und einem daran in Längsrichtung verstellbaren Arm (38), wobei an jedem Arm ein Träger (34) eines Aufnahmehohlkörpers für einen der Verlängerungsstäbe (18) angelenkt ist, und die Gelenkachsen (39) etwa in der durch die Arme (37, 38) definierten Ebene derart schräg verlaufen, daß sie sich in der Verlängerung auf der dem Längsholm (36) zugewandten Seite kreuzen.

7. Implantatsystem nach Anspruch 6, dadurch gekennzeichnet, daß die Gelenkachsen (39) einen Winkel von etwa 30° bezüglich des Längsholms (36) aufweisen.

8. Implantatsystem nach Anspruch 6 in Kombination mit einem Traktionsinstrument mit zwei von einer Spindel (30) durchsetzten und durch deren Schraubbewegung zusammenziehbare Hülsen (32, 28), von denen eine einen Haken (33) und die andere einen zur Zugrichtung schrägen Arm (29) aufweist, an dem ein Aufnahmehohlkörper für einen der Verlängerungsstäbe (18) so angebracht ist, daß er um eine zur Gewindespindel (30) senkrechte Achse einstell- und fixierbar ist.

9. Implantatsystem nach Anspruch 8, dadurch gekennzeichnet, daß der Längsholm (36) des Distraktionsinstruments eine Zahnstange ist, deren Zähne an der von den Armen (37, 38) abgewandten Seite liegen und insbesondere hinsichtlich ihres Abstandes so beschaffen sind, daß die Haken (33) der Traktionsinstrumente zwischen die Zähne eingelegt werden können.

## Claims

1. Implant for stabilising the spinal column with a longitudinal support in the form of a profile rod (1), two cross arms (2), which may be clamped thereto, and anchoring screws (3), which may be secured to the cross arms on an adjustable inclination, wherein the cross arms (2) each comprise a clamping piece (5, 20) and a connector (12, 25) and the connectors each have a grid-like surface (26) with a central hole for screw attachment of an anchoring screw at a fixed angle, characterised in that the clamping pieces and the connectors are screwed into one another; and that the anchoring screws (3) each have a flattened screw head, onto which a respective extension rod (18) may be attached, its thickened end section having a trough-shaped recess (19).

2. Implant according to Claim 1, characterised in that the clamping piece has two pincer-like jaws (6, 7) which form a receiving opening (8) to fit the profile rod (1) and which are drawn together by means of a clamping screw (9); and that a threaded hole (10) is provided on the opposite side of the receiving opening on the clamping piece to screw the connector (12) vertically to the receiving opening (8).

3. Implant according to Claim 2, characterised in that a jaw (21) is articulated in the manner of a hinge to the clamping piece (20).

4. Implant according to Claim 1, characterised in that the clamping piece (5, 20) has a bent form so that the axis of the threaded hole (10) and the clamping screw (9) stand at an oblique angle to one another.

5. Implant according to Claim 4, characterised in that the axes of the threaded hole (10) and the clamping screw (9) are displaced relative to one another in the direction of the profile rod (1) on the clamping piece.

6. Implant system, characterised by an implant according to Claim 1 in combination with a tensioning instrument with a longitudinal bar (36), an arm (37) fastened thereto and an arm (38) which may be adjusted thereon in longitudinal direction, wherein a support (34) of a hollow member for receiving one of the extension rods (18) is articulated to each arm, and the pivot axes (39) run approximately in the plane defined by the arms (37, 38) at such an oblique angle that they intersect in the extension to the side facing the longitudinal bar (36).

7. Implant system according to Claim 6, characterised in that the pivot axes (39) are at an angle of about 30° with respect to the longitudinal bar (36).

8. Implant system according to Claim 6 in combination with a traction instrument having two sleeves (32, 38), which have a spindle (30) passing through them and may be drawn together by its screwing movement, one of said sleeves having a hook (33) and the other having an arm (29) at an angle to the direction of traction, on which a hollow member for receiving one of the extension rods (18) is attached so that it may be adjusted or fixed around an axis vertical to the threaded spindle (30).

9. Implant system according to Claim 8, characterised in that the longitudinal bar (36) of the tensioning instrument is a toothed rod, the teeth of which lie on the side facing away from the arms (37, 38) and are constructed in particular with respect to their spacing so that the hooks (33) of the traction instrument can be inserted between the teeth.

## Revendications

1. Implant pour la stabilisation de la colonne vertébrale, comportant un support longitudinal sous forme d'une barre profilée (1), des bras transversaux (2) pouvant être fixés sur celle-ci, et des vis d'ancrage (3) pouvant être fixées aux bras transversaux avec une inclinaison réglable, les bras transversaux (2) étant constitués, à chaque fois, d'une pièce de serrage (5,20) et d'une attache (12,25) et les attaches comportant, à chaque fois, une surface crantée (26) ayant un perçage central, pour visser, sous un angle fixe, une vis d'ancrage (3),
caractérisé en ce que les pièces de serrage et les attaches sont vissées les unes dans les autres, et en ce que les vis d'ancrage (3) présentent, à chaque fois, une tête de vis aplatie, sur laquelle peut être montée, à chaque fois, une barre de prolongement (18) ayant un évidement (19) en forme de cuvette de son tronçon d'extrémité épaissi.

2. Implant selon la revendication 1,
caractérisé en ce que la pièce de serrage présente deux mâchoires (6,7) du type pince, qui forment une ouverture de réception adaptée (8) pour la barre profilée (1) et sont serrées au moyen d'une vis de serrage (9), et en ce que, sur le côté opposé de l'ouverture de réception sur la pièce de serrage, il est prévu un perçage taraudé (10) pour visser l'attache (12) perpendiculairement à l'ouverture de réception (8).

3. Implant selon la revendication 2,
caractérisé en ce qu'une mâchoire (21) est articulée, à la manière d'une charnière, à la pièce de serrage (20).

4. Implant selon la revendication 1,
caractérise en ce que la pièce de serrage (5,20) présente une forme courbée, de sorte que les axes du perçage taraudé (10) et de la vis de serrage (9) forment, l'un par rapport à l'autre, un angle oblique.

5. Implant selon la revendication 4,
caractérisé en ce que les axes du perçage taraudé (10) et de la vis de serrage (9) sont décalés l'un par rapport à l'autre sur la pièce de serrage en direction de la barre profilée (1).

6. Système d'implant,
caractérisé par un implant selon la revendication 1, en combinaison avec un instrument de maintien ayant un longeron (36), un bras (37) fixé à celui-ci, et un bras (38) réglable en direction longitudinale sur celui-ci, un support (34) d'un corps creux de réception pour une des barres de prolongement (18) étant articulé à chaque bras, et les axes d'articulation (39) s'étendant de façon inclinée à peu près dans le plan défini par les bras (37,38), de sorte qu'ils se croisent dans le prolongement sur le côté en regard du longeron (36).

7. Système d'implant selon la revendication 6,
caractérisé en ce que les axes d'articulation (39) présentent un angle d'environ 30° par rapport au longeron (36).

8. Système d'implant selon la revendication 6, en combinaison avec un instrument de traction comportant deux manchons (32,28) pouvant être serrés par leur mouvement de vissage et traversés par une tige (30), desquels manchons l'un présente un crochet (33) et l'autre un bras (29) incliné par rapport à la direction de traction, auquel est appliqué un corps creux de réception pour une des barres de prolongement (18), de sorte qu'il peut être fixé et réglé autour d'un axe perpendiculaire à la tige filetée (30).

9. Système d'implant selon la revendication 8,
caractérisé en ce que le longeron (36) de l'instrument de maintien est une crémaillère dont les dents se trouvent sur le côté opposé aux bras (37,38) et sont réalisées, en particulier relativement à leur écartement, de sorte que les crochets (33) des instruments de traction peuvent être introduits entre les dents.
